# EUROPEAN PATENT SPECIFICATION

(11) **EP 1 415 591 B1**
(45) Date of publication and mention of the grant of the patent: **12.04.2006**
(21) Application number: 03256723.2
(22) Date of filing: 23.10.2003
(51) Int. Cl.: A61B 5/053

(54) **Apparatus and method for measuring local skin impedance using multiple electrodes array**
Gerät und Verfahren zur Messung der örtlichen Hautimpedanz mit einer Mehrfachelektrodenanordnung
Dispositif et procédé pour mesurer l'impédance locale de la peau en utilisant des réseaux d'électrodes multiples

(30) Priority: 24.10.2002 KR 2002065185
(43) Date of publication of application: 06.05.2004
(73) Proprietor: SAMSUNG ELECTRONICS CO., LTD., Suwon-City, Kyungki-do (KR)
(72) Inventor: Shin, Sang-hoon, Bundang-gu Seongnam-city Kyungki-do (KR); Park, Jun-hyub, Bundang-gu Seongnam-city Kyungki-do (KR); Lim, Geun-bae, Paldal-gu Suwon-city Kyungki-do (KR); Jang, Woo-young, Gangnam-gu Seoul (KR); Gi, Ho-seong Samsung Advanced Institute of Techno., Yongin-city Kyungki-do (KR)
(74) Representative: Greene, Simon Kenneth

(56) References cited:
- US-A- 4 182 314
- MARTINSEN O G ET AL: "2D skin admittance mapping" ENGINEERING IN MEDICINE AND BIOLOGY SOCIETY, 1994. ENGINEERING ADVANCES: NEW OPPORTUNITIES FOR BIOMEDICAL ENGINEERS., PROCEEDINGS OF THE 16TH ANNUAL INTERNATIONAL CONFERENCE OF THE IEEE BALTIMORE, MD, USA 3-6 NOV. 1994, NEW YORK, NY, USA,IEEE, US, 3 November 1994 (1994-11-03), pages 892-893, XP010145693 ISBN: 0-7803-2050-6
- FUKUMOTO T ET AL: "A basic study about multi channel measurment of skin impedance vector loci on the acupunctur points" PROCEEDINGS OF THE 23RD. ANNUAL INTERNATIONAL CONFERENCE OF THE IEEE ENGINEERING IN MEDICINE AND BIOLOGY SOCIETY. 2001 CONFERENCE PROCEEDINGS. (EMBS). INSTANBUL, TURKEY, OCT. 25 - 28, 2001, ANNUAL INTERNATIONAL CONFERENCE OF THE IEEE ENGINEERING IN M, vol. 1 OF 4. CONF. 23, 25 October 2001 (2001-10-25), pages 3392-3395, XP010593835 ISBN: 0-7803-7211-5
- KWOK G ET AL: "MAPPING ACUPUNCTURE POINTS USING MULTI CHANNEL DEVICE" AUSTRALASIAN PHYSICAL AND ENGINEERING SCIENCES IN MEDICINE, ASTRALASIAN COLLEGE OF PHYSICAL SCIENTISTS IN, AU, vol. 21, no. 2, 1998, pages 68-72, XP000911092 ISSN: 0158-9938
- YAMAMOTO Y ET AL: "DYNAMIC SYSTEM FOR THE MEASUREMENT OF ELECTRICAL SKIN IMPEDANCE" MEDICAL AND BIOLOGICAL ENGINEERING AND COMPUTING, PETER PEREGRINUS LTD. STEVENAGE, GB, vol. 17, no. 1, January 1979 (1979-01), pages 135-137, XP001173002 ISSN: 0140-0118

## Description

The present invention relates to the measurement of an electrical impedance of a vital component of a human body, such as the skin. More particularly, the present invention relates to an apparatus and method for measuring a local impedance distribution in human skin using multiple array electrodes.

A variety of types of information related to a human body can be obtained based on a local impedance distribution in the human skin. For example, a local impedance distribution has been used for a urea test, a blood count test, and a blood coagulation test. In addition, the local impedance distribution in the human skin can be used to measure skin conductivity to determine an optimal position to which an electrode should be attached or to test a blood coagulation status in a blood vessel to diagnose myocardial infarction or sclerosis of the arteries.
Disadvantages of conventional approaches include difficulty in accurately detecting a local impedance distribution and trend in the skin and a failure to consider basic contact resistance problems.

A measuring system, based on a vehicle with 16 electrodes, for the investigation of local variations in skin electrical admittance with a 2D resolution of 1 mm is disclosed in "2D Skin Admittance Mapping" by Martinsen et al (16^{th} Annual International Conference of the IEEE, NY, 3 November 1994. Pages 892-893, XP010145693, ISBN: 0-7803-2050-6).

The present invention provides an apparatus and method for measuring local skin impedance to accurately determine a position of an acupuncture point on the human skin.

According to an aspect of the present invention, there is provided an apparatus for measuring local skin impedance according to claim 1.

According to another aspect of the present invention, there is provided a method of acquiring a local skin impedance according to claim 11.

According to yet another aspect of the present invention, there is provided a computer program according to claim 17.

Preferably, the multi-channel electrode includes a plurality of measurement sensors arranged in a matrix shape on an electrode surface having a predetermined area.

According to yet another feature of an embodiment of the present invention, there is provided a computer readable medium having embodied therein a computer program for the methods according to the present invention.

The above and other features and advantages of the present invention will become more apparent to those of ordinary skill in the art by describing in detail preferred embodiments thereof with reference to the attached drawings in which:
FIG. 1 is a schematic diagram of an apparatus for measuring local skin impedance, according to an embodiment of the present invention;
FIGS. 2 and 3 illustrate an end view and a side view, respectively, of a multi-channel electrode according to an embodiment of the present invention;
FIG. 4 illustrates an example of a procedure for measuring skin impedance using an apparatus for measuring local skin impedance according to an embodiment of the present invention;
FIG. 5 is a flowchart of a clinical demonstration procedure in which a tester measures a patient's skin impedance using an apparatus for measuring local skin impedance according to an embodiment of the present invention;
FIG. 6 is a flowchart of a method of measuring local skin impedance using the apparatus shown in FIG. 1;
FIG. 7 illustrates various pressures applied to the multi-channel electrode when skin impedance is measured using an apparatus for measuring local skin impedance according to an embodiment of the present invention, and states of the multi-channel electrode depending on the pressures;
FIG. 8 illustrates an example in which skin impedance is measured at different pressures on the multi-channel electrode using an apparatus for measuring local skin impedance according to an embodiment of the present invention;
FIGS. 9A-9D illustrate two- and three-dimensional distributions of skin impedance at Zusanli when a weak pressure is applied to the multi-channel electrode shown in FIG. 8;
FIGS. 10A-10D illustrate two- and three-dimensional distributions of skin impedance at Zusanli when a medium pressure is applied to the multi-channel electrode shown in FIG. 8;
FIGS. 11A-11C illustrate two- and three-dimensional distributions of skin impedance at Zusanli when a strong pressure is applied to the multi-channel electrode shown in FIG. 8;
FIG. 12 illustrates an example in which skin impedance is measured on a governor vessel using the apparatus shown in FIG. 1; and
FIGS. 13A-13C illustrate two- and three-dimensional distributions of the skin impedance on the governor vessel, which is acquired using a multi-channel electrode shown in FIG. 12.

The present invention will now be described more fully hereinafter with reference to the accompanying drawings, in which preferred embodiments of the invention are shown. The invention may, however, be embodied in different forms and should not be construed as limited to the embodiments set forth herein. Rather, these embodiments are provided so that this disclosure will be thorough and complete, and will fully convey the scope of the invention to those skilled in the art. Like reference numerals refer to like elements throughout.

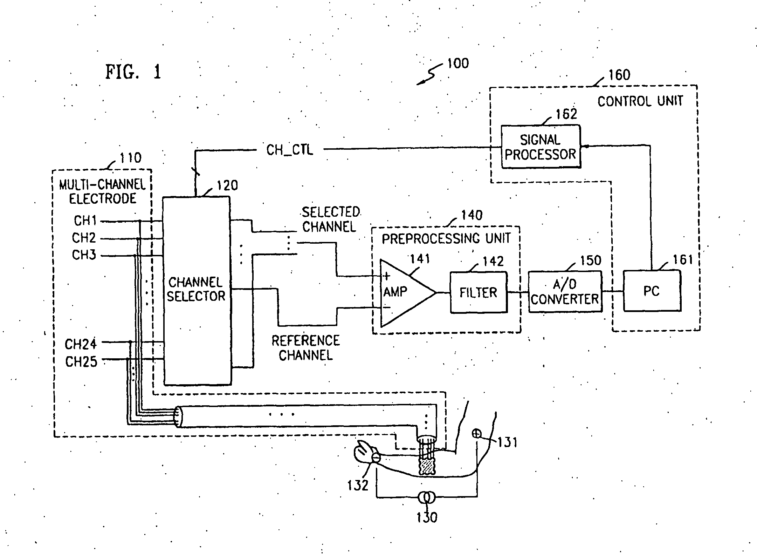

FIG. 1 is a schematic diagram of an apparatus 100 for measuring local skin impedance, according to an embodiment of the present invention. Referring to FIG. 1, the apparatus 100 includes a multi-channel electrode 110, a channel selector 120, a constant current source 130, a preprocessing unit 140, an analog-to-digital (A/D) converter 150, and a control unit 160.

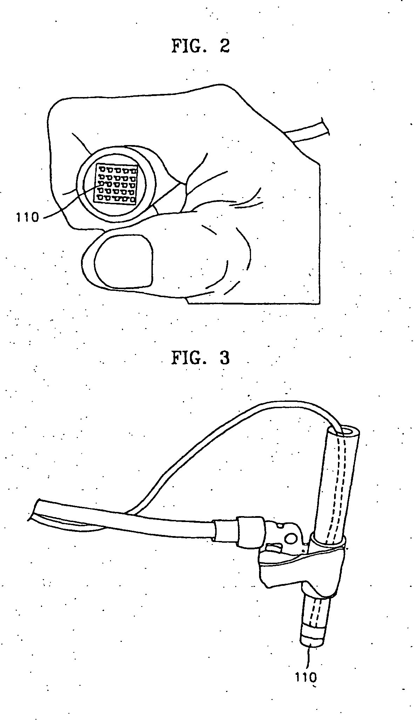

The multi-channel electrode 110 includes a plurality of measurement sensors arranged in a matrix shape on an electrode surface having a predetermined area and is used to measure skin impedance in a very small area (i.e., a local zone). A more detailed description of the structure of the multi-channel electrode 110 will be described with reference to FIGS. 2 and 3.

FIGS. 2 and 3 illustrate an end view and a side view, respectively, of the multi-channel electrode 110 according to an embodiment of the present invention. Referring to FIGS. 2 and 3, the plurality of measurement sensors in the multi-channel electrode 110 are implemented by leeno pins having a height of about 1 mm and are arranged at regular intervals on the electrode surface at an end of a cylindrical probe rod having a diameter of about 10 mm. The leeno pins are measurement sensors manufactured by Leeno Industrial Inc. These pins have excellent tension due to a spring and are made of a metal conductor so as to be suitable for automation.

As shown in the drawings, in order to minimize a patient's pain due to a contact pressure during a local skin impedance measurement, the measurement sensors have a pin protruding by a short length of about 1 mm. A head portion of the multi-channel electrode 110 is structured to be easily separated from the cylindrical probe rod so that the measurement sensors, i.e., leeno pins, can be easily replaced. During a local skin impedance measurement, pressure applied to each of the measurement sensors of the multi-channel electrode 110 can be uniformly controlled, or can be controlled to be different for each measurement sensor, depending on the curvature of a measured body part.

In FIGS. 1 and 2, the multi-channel electrode 110 includes twenty-five (25) measurement sensors arranged in a 5 x 5 matrix and twenty-five (25) channels. However, more than twenty-five (25) channels may be included in the multi-channel electrode 110. Additionally, a micro-electro-mechanical system (MEMS) electrode, instead of the leeno pins, may be used.

Referring back to FIG. 1, the channel selector 120 selects each of the channels in response to a channel control signal CH_CTL generated by a signal processor 162 included in the control unit 160. The apparatus 100 sequentially measures a skin potential at each of the channels selected by the channel selector 120 until measurement at all of the channels of the multi-channel electrode 110 is completed.

The constant current source 130 supplies a constant current to a body part in order to measure the skin potential. A predetermined current output from the constant current source 130 is applied to the skin through a positive (+) electrode 131, then output to a negative (-) electrode 132, and then flows back into the constant current source 130. The positive and negative electrodes 131 and 132 are each attached to a point on the skin. Here, the multi-channel electrode 110 measures a skin impedance between the positive electrode 131 and the negative electrode 132 between which the constant current flows. Since a predetermined current flows in the body part, the skin impedance is obtained using a potential value acquired at each channel of the multi-channel electrode 110.

The preprocessing unit 140 includes a differential amplifier (AMP) 141 and a filter 142. While the predetermined current flows through a measured region due to the operation of the constant current source 130, a potential value acquired at each channel of the multi-channel electrode 110 is amplified by the AMP 141. The amplified potential value is filtered by the filter 142. It is preferable that the AMP 141 has high Common Mode Rejection characteristic and low noise characteristic such as, for example, a AD620 made by Analog Devices. The filter 142 may be implemented by a sixth-order Butterworth filter having a cut-off frequency of 4 Hz or less. A battery (not shown) is used as a power supply source of the preprocessing unit 140.

The A/D converter 150 receives an analog signal output from the preprocessing unit 140 and converts the analog signal into a digital signal so that the signal can be processed in a computer.

The control unit 160 includes a personal computer (PC) 161 that controls the entire apparatus 100, and a signal processor 162, which processes a signal acquired from the multi-channel electrode 110 under the control of the PC 161.

The digital potential value of each channel input from the A/D converter 150 is input to the signal processor 162 through the PC 161. The signal processor 162 may use the Laboratory Virtual Instrument Engineering Workbench (LabVIEW) software manufactured by the National Instruments in order to facilitate connection to the PC 161. The LabVIEW software is an analysis software system, which makes it possible to perform a measurement generally performed by an instrument such as an oscilloscope using a PC, and expresses the potential values acquired by the multi-channel electrode 110 as a two- and three-dimensional spatial impedance distribution. While the LabVIEW software may be used in the signal processor 162, this is just an example and other newly developed software or hardware systems can be used in the signal processor 162.

As described above, the apparatus 100 for measuring local skin impedance according to the present invention measures an electrical impedance component induced by the current applied between two points 131 and 132 on the skin. The apparatus 100 acquires skin impedance using the multi-channel electrode 110, which can be applied to a local region, and analyzes the acquired skin impedance in diverse ways so that the position of each acupuncture point on the human skin can be accurately determined. The following description concerns a method of measuring skin impedance using the apparatus 100 for measuring local skin impedance, according to an embodiment of the present invention.

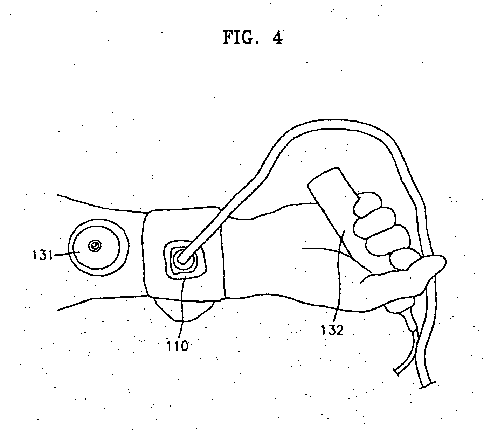

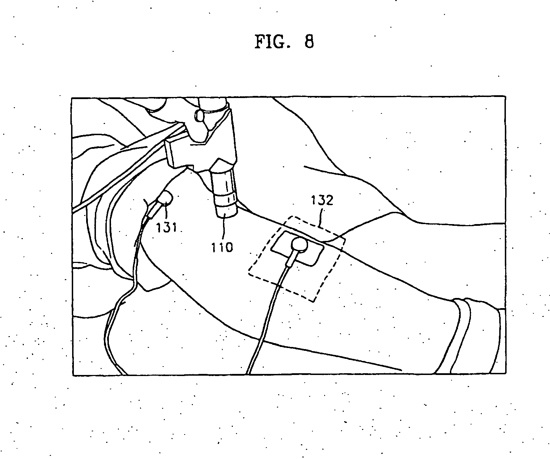

FIG. 4 illustrates an example of a procedure for acquiring skin impedance using the apparatus 100 for measuring local skin impedance. Referring to FIG. 4, the positive and negative electrodes 131 and 132 of the constant current source 130 are separated by a predetermined distance on opposite sides of a measured region at which the multi-channel electrode 110 is placed. Here, the positive electrode 131 is implemented by an electrocardiogram (ECG) electrode. The negative electrode 132 is implemented by a brass electrode so that a patient can hold the negative electrode 132 in the patient's hand. When the patient cannot hold the negative electrode 132, an electrode having a wide contact surface can be used as the negative electrode 132 so as to be attached to a predetermined position, as shown in FIG. 8.

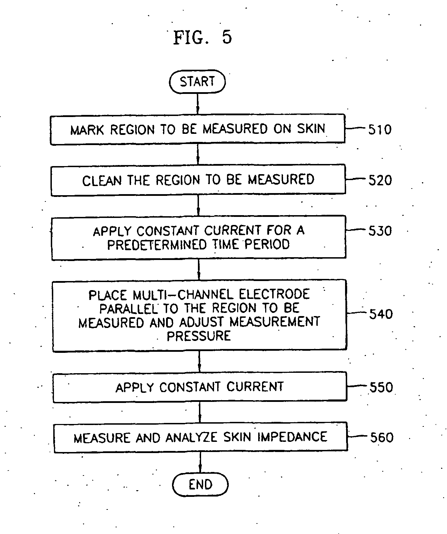

FIG. 5 is a flowchart of a clinical demonstration procedure in which a tester measures a patient's skin impedance using the apparatus 100 for measuring local skin impedance, according to an embodiment of the present invention. Referring to FIG. 5, in step 510, a region to be measured is marked on the patient's skin. In step 520, the region to be measured is cleaned with alcohol soaked cotton to remove foreign substances and to maintain a degree of hydration of the skin during the measurement.

Subsequently, in step 530, as illustrated in FIG. 4, the two electrodes 131 and 132 of the constant current source 130 are disposed to be separated from the region to be measured by a predetermined distance and a constant current is applied to the region to be measured through the two electrodes 131 and 132 for a predetermined time period. Next, in step 540, the multi-channel electrode 110 is placed parallel to the region to be measured, and a measurement pressure is adjusted. In operation, a pressure applied to the multi-channel electrode 110 during measurement exerts a significant influence on a measured value. Accordingly, it is necessary to maximize the pressure on the entire surface of the region to be measured without causing the patient to experience pain.

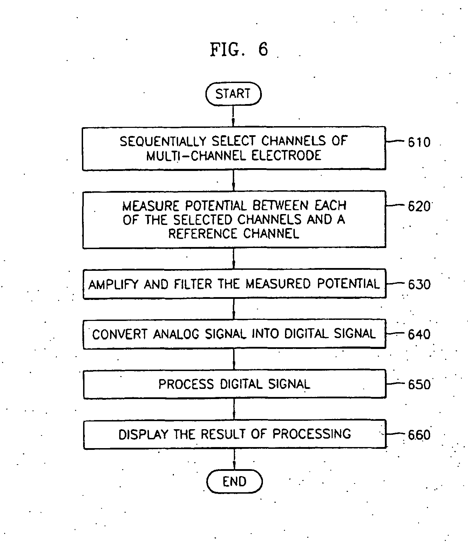

After the multi-channel electrode 110 is set up in step 540, in step 550, the constant current is applied between the two electrodes 131 and 132 from the constant current source 130. While the constant current is applied, in step 560, a skin impedance is measured, and the result of measurement is analyzed, thereby determining the correct position of an acupuncture point. The following description concerns the operation of the apparatus 100 for measuring a local skin impedance. FIG. 6 is a flowchart of a method of measuring local skin impedance using the apparatus 100 shown in FIG. 1. Referring to FIG. 6, in step 610, the apparatus 100 sequentially selects the channels of the multi-channel electrode 110 through the channel selector 120 and then, in step 620, measures a potential between each of the selected channel and a reference channel.

Thereafter, in step 630, the preprocessing unit 140 amplifies the measured potential using the AMP 141 and filters the amplified potential using the filter 142. Subsequently, in step 640, an analog output of the preprocessing unit 140 is input into the A/D converter 150 and is converted into a digital signal by the A/D converter 150. In step 650, the digital signal output from the A/D converter 150 is input into and processed by the signal processor 162. In step 660, the result of the processing, i.e., a spatial distribution of skin impedance, is displayed in two and three dimensions. The tester is able to determine the position of an acupuncture point based on the results of the processing and is able to analyze the characteristics of the acupuncture point.

A pressure applied to the multi-channel electrode 110 during the test significantly influences the result of measurement. Accordingly, during the clinical demonstration using the apparatus 100, three types of pressures, i.e., a weak pressure, a medium pressure, and a strong pressure, were applied to the multi-channel electrode 110.

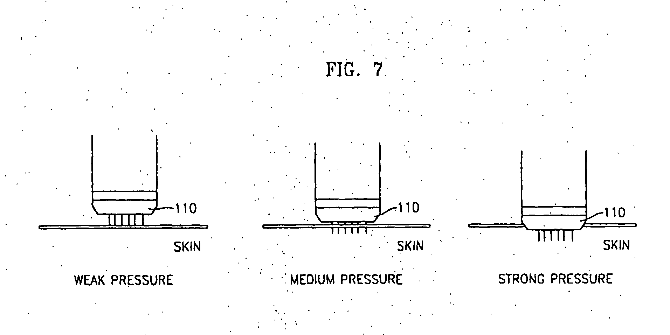

FIG. 7 illustrates the various pressures applied to the multi-channel electrode 110 when a skin impedance is measured using the apparatus 100 for measuring local skin impedance and states of the multi-channel electrode 110 depending on the various pressures. Referring to FIG. 7, when a weak pressure is applied to the multi-channel electrode 110, the multi-channel electrode 110 just contacts the skin. When a medium pressure is applied, the multi-channel electrode 110 slightly presses the skin. When a strong pressure is applied, the multi-channel electrode 110 maximally presses the skin without causing a patient to experience any pain. The following description concerns the results of experiments in which each of the pressures is applied to the multi-channel electrode 110.

FIG. 8 illustrates an example in which skin impedance is measured at each of the pressures on the multi-channel electrode 110 using the apparatus 100 for measuring local skin impedance, according to an embodiment of the present invention. Referring to FIG. 8, an acupuncture point Zusanli is located in a region that is flatter than the regions where other acupuncture points are located. In this situation, since a region to be measured is below the knee, it is preferable to use an ECG electrode having a large contact portion as the negative electrode 132 of the constant current source 130 rather than the brass electrode as shown in FIG. 4. The ECG electrode is attached to be separated from the Zusanli point by a predetermined distance and is used as the negative electrode 132.

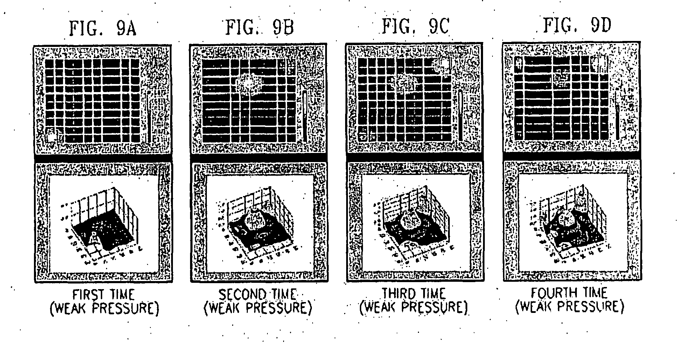

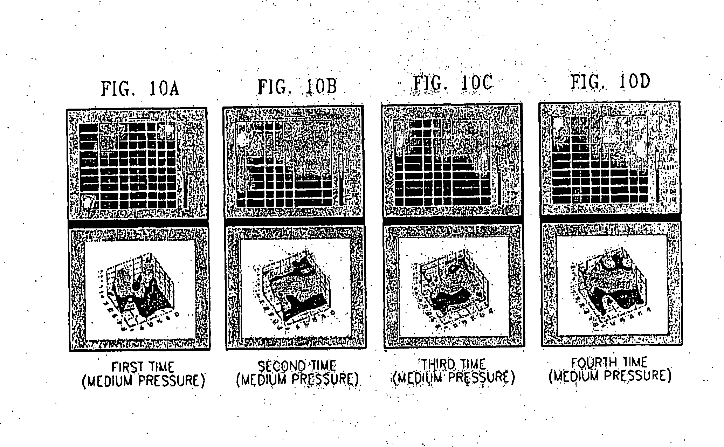

FIGS. 9A-9D illustrate two- and three-dimensional distributions of a skin impedance at the Zusanli point when a weak pressure was applied to the multi-channel electrode 110 shown in FIG. 8. FIGS. 10A-10D illustrate two- and three-dimensional distributions of a skin impedance at the Zusanli point when a medium pressure was applied to the multi-channel electrode 110 shown in FIG. 8.

Referring to FIGS. 9A-9D and 10A-10D, when the weak pressure was applied to the multi-channel electrode 110, a region in which a potential difference increases extends in time. This indicates that the multi-channel electrode 110 was gradually pressed down, and thus the pressure applied to the multi-channel electrode 110 was gradually increased in time. Accordingly, it is apparent that the result of measurement may change depending on the pressure applied to the multi-channel electrode 110.

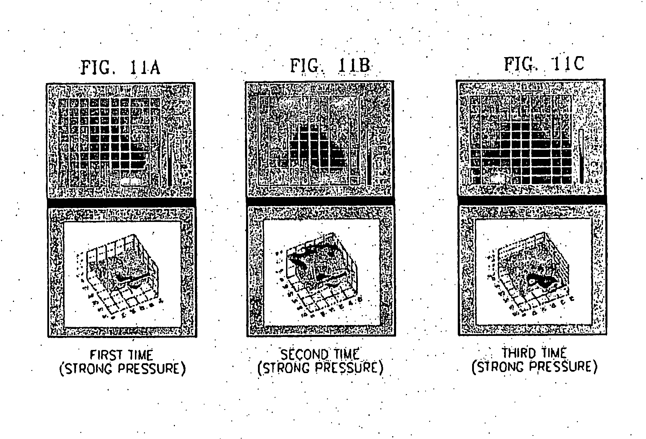

FIGS. 11A-11C illustrate two- and three-dimensional distributions of a skin impedance at the Zusanli point when a strong pressure was applied to the multi-channel electrode 110 shown in FIG. 8. Referring to FIGS. 11A-11C, when the strong pressure was applied to the multi-channel electrode 110, the distinct impedance distribution at the acupuncture point was similar for several trials, unlike the results of the Zusanli measurement shown in FIGS. 9A-9D and 10A-10D. Specifically, when the constant current was applied to the measured region, a potential value was lower, i.e., a conductibility was higher and a resistance was lower, at the Zusanli acupuncture point than at non-acupuncture points around the Zusanli acupuncture point. Such a low resistance characteristic of the Zusanli also appears at other acupuncture points, for example, a governor vessel.

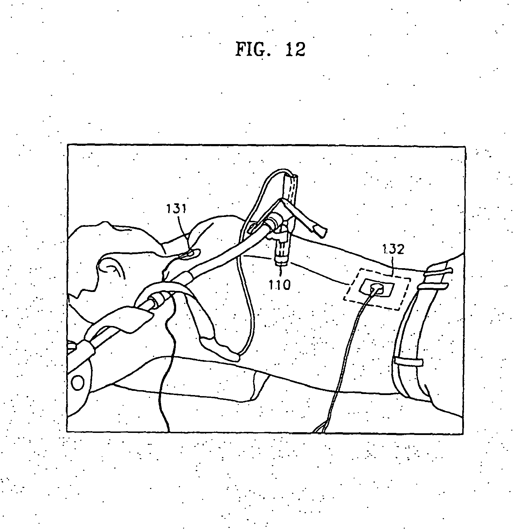

FIG. 12 illustrates an example in which skin impedance is measured on the governor vessel using the apparatus 100 shown in FIG. 1. Referring to FIG. 12, it is preferable to use an ECG electrode having a large contact portion than using a brass electrode, as the negative electrode 132 among the two electrodes 131 and 132 of the constant current source 130. The ECG electrode is attached to be separated from the governor vessel by a predetermined distance and is used as the negative electrode 132.

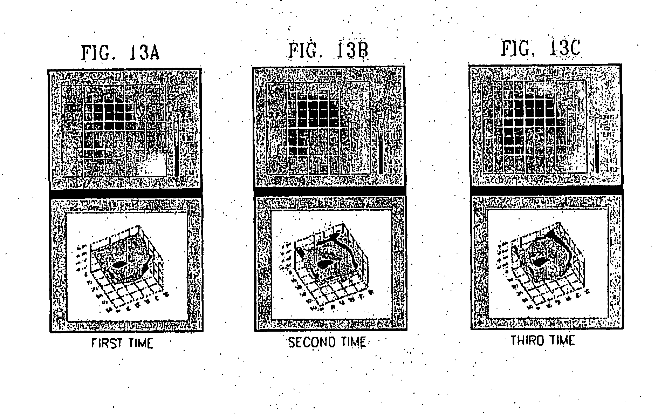

FIGS. 13A-13C illustrates two- and three-dimensional distributions of a skin impedance on the governor vessel, which is measured through the multi-channel electrode 110 shown in FIG. 12. Similar to the results of the measurement shown in FIGS. 11A-11C, the distinct impedance distribution at this acupuncture point was similar for several trials.

The above-described preferred and exemplary embodiments of the present invention may be embodied as computer programs and may also be embodied in a general-purpose digital computer for executing the computer programs using a computer readable medium. The computer readable medium may include storage media, such as, magnetic storage media (e.g., ROMs, floppy discs, hard discs, and the like), optically readable media (e.g., CD-ROMs, DVDs, and the like), and carrier waves (transmission over the Internet).

As described above, an apparatus for measuring local skin impedance according to the present invention is able to measure a skin impedance distribution at a local region using a multi-channel electrode and accurately analyze the measured skin impedance distribution. Accordingly, a position of an acupuncture point on a human body can be easily found out within a very small error range. In addition, the characteristics of each meridian system, i.e., a group of acupuncture points, can be analyzed and used in diagnosing and treating human diseases.

Preferred embodiments of the present invention have been disclosed herein and, although specific terms are employed, they are used and are to be interpreted in a generic and descriptive sense only and not for purpose of limitation.

## Claims

1. An apparatus for measuring local skin impedance, comprising:
a multi-channel electrode (110) including a plurality of measurement sensors on an electrode surface having a predetermined area;
a channel selector (120) arranged to select each of channels included in the multi-channel electrode (110) in response to a channel control signal;
a preprocessing unit (140) arranged to amplify and filter a potential value measured at each of the channels while the predetermined constant current is flowing through the region to be measured;
an analog-to-digital converter (150) arranged to convert the potential value output from the preprocessing unit (140) into a digital signal; and **characterised by**:
a constant current source (130) arranged to apply a predetermined constant current to a region to be measured;
a control unit (160) arranged to generate the channel control signal, for processing the digital signal output from the analog-to-digital converter (150), and to control the entire apparatus; and
wherein the apparatus is arranged to control the pressure applied to each of the measurement sensors so that the pressure applied by the measurement sensors can be varied.

2. The apparatus as claimed in claim 1, wherein the plurality of measurement sensors is arranged in a matrix shape on the electrode surface.

3. The apparatus as claimed in claim 1 or 2, wherein the measurement sensors are pin electrodes made of a metal conductor and include a spring.

4. The apparatus as claimed in any preceding claim, wherein the multi-channel further comprises twenty-five (25) measurement sensors arranged in a 5 x 5 matrix.

5. The apparatus as claimed in any preceding claim, wherein the multi-channel electrode (110) comprises a micro-electro-mechanical system (MEMS) electrode.

6. The apparatus as claimed in any preceding claim, wherein the constant current source (130) comprises:
a positive electrode (131) and a negative electrode (132), arranged to be attached to a location on skin centering around the region to be measured such that the positive and negative electrodes are separated from the region to be measured by a predetermined distance, so that the predetermined constant current output from the constant current source (130) is applied to the skin through the positive electrode (131), then output from the skin through the negative electrode (132), and then flows back in the constant current source (130).

7. The apparatus as claimed in any preceding claim, wherein the preprocessing unit (140) comprises:
a differential amplifier (141); and
a filter (142).

8. The apparatus as claimed in claim 7, wherein the filter (142) is a sixth-order Butterworth filter having a cut-off frequency of 4 Hz or less.

9. The apparatus as claimed in any preceding claim, wherein the control unit (160) comprises:
a personal computer (161) for controlling the apparatus; and
a signal processor (162) for generating the channel control signal and expressing the potential values acquired at each of the channels of the multi-channel electrode (110) as a two-dimensional impedance distribution and a three-dimensional impedance distribution under a control of the personal computer (161).

10. The apparatus as claimed in any preceding claim, wherein the control unit (160) determines an acupuncture point position using the digital potential values.

11. A method of acquiring a local skin impedance, comprising:
(a) disposing two electrodes of a constant current source (130) centering around a region to be measured on a patient's skin to be separated from the region to be measured by a predetermined distance and applying a predetermined constant current to the skin through the two electrodes for a predetermined time period;
(b) positioning a multi-channel electrode (110) parallel to the region to be measured and controlling the pressure applied to each of the measurement sensors of the multi-channel electrode (110) so that the pressure applied by the measurement sensors can be varied; and
(c) applying the predetermined constant current between the two electrodes of the constant current source (130) and measuring skin impedance at the region to be measured while the predetermined constant current is being applied.

12. A method as claimed in claim 11, further comprising:
measuring a potential value at each of a plurality of channels included in a multi-channel electrode (110) disposed between two electrodes of a constant current source (130) for applying a predetermined constant current to a patient's skin through the two electrodes;
amplifying and filtering the potential value at each channel;
converting the filtered potential value from an analog format into a digital format; and
analyzing the potential value in the digital format and displaying the results of the analysis in a form of a spatial impedance distribution in two and three dimensions.

13. The method as claimed in claim 11 or 12, wherein the multi-channel electrode (110) comprises:
a plurality of measurement sensors arranged in a matrix shape on an electrode surface having a predetermined area.

14. The method as claimed in claim 11, 12 or 13, wherein in (b), the pressure applied to each of the measurement sensors is adjusted depending on a curvature of the region to be measured during measurement of skin impedance.

15. The method as claimed in any of claims 12 to 14, wherein the channels are selected in turn.

16. The method as claimed in any claims 11 to 15, wherein the potential in each channel is measured, filtered and amplified while the constant current is flowing.

17. A computer program comprising computer program code means adapted to cause the apparatus according to claims 1-10 to perform all the steps of any of claims 11 to 16

18. A computer program as claimed in claim 17 embodied on a computer readable medium.

## Revendications

1. Appareil de mesure de l'impédance locale de la peau, comprenant :
une électrode à canaux multiples (110) comprenant une pluralité de capteurs de mesure sur une surface d'électrode ayant une aire prédéterminée ;
un sélecteur de canal (120) arrangé pour choisir chacun des canaux inclus dans l'électrode à canaux multiples (110) en réponse à un signal de contrôle de canal ;
une unité de prétraitement (140) arrangée pour amplifier et filtrer une valeur de potentiel mesurée à chacun des canaux alors que le courant constant prédéterminé circule dans la région à mesurer ;
un convertisseur analogique-numérique (150) arrangé pour convertir la sortie de la valeur de potentiel de l'unité de prétraitement (140) en un signal numérique ; et **caractérisé par** :
une source de courant constant (130) arrangée pour appliquer un courant constant prédéterminé à une région à mesurer ;
une unité de contrôle (160) arrangée pour générer le signal de contrôle du canal, pour traiter la sortie de signal numérique du convertisseur analogique-numérique (150), et pour contrôler la totalité de l'appareil ; et
dans lequel l'appareil est arrangé pour contrôler la pression appliquée à chacun des capteurs de mesure de manière à ce que la pression appliquée par les capteurs de mesure puisse varier.

2. Appareil selon la revendication 1, dans lequel la pluralité de capteurs de mesure est arrangée en une forme de matrice sur la surface de l'électrode.

3. Appareil selon la revendication 1 ou 2, dans lequel les capteurs de mesure sont des électrodes à broche formées par un conducteur métallique et comprennent un ressort.

4. Appareil selon l'une quelconque des revendications précédentes, dans lequel le canal multiple comprend en outre vingt-cinq (25) capteurs de mesure arrangés en une matrice 5 x 5.

5. Appareil selon l'une quelconque des revendications précédentes, dans lequel l'électrode à canaux multiples (110) comprend une électrode à microsystème électromécanique (MEMS).

6. Appareil selon l'une quelconque des revendications précédentes, dans lequel la source de courant constant (130) comprend :
une électrode positive (131) et une électrode négative (132), qui sont fixées à un emplacement sur la peau centré autour de la région à mesurer de telle manière que les électrodes positive et négative soient séparées de la région à mesurer par une distance prédéterminée, de telle sorte qu'une sortie de courant prédéterminée de la source de courant constant (130) est appliquée à la peau par une électrode positive (131), puis sort de la peau par l'électrode négative (132), et retourne dans la source de courant constant (130).

7. Appareil selon l'une quelconque des revendications précédentes, dans lequel l'unité de prétraitement (140) comprend :
un amplificateur différentiel (141) ; et
un filtre (142).

8. Appareil selon la revendication 7, dans lequel le filtre (142) est un filtre Butterworth de sixième ordre ayant une fréquence de coupure de 4 Hz ou moins.

9. Appareil selon l'une quelconque des revendications précédentes, dans lequel l'unité de contrôle (160) comprend :
un ordinateur personnel (161) pour contrôler l'appareil ; et
un processeur de signal (162) pour générer le signal de contrôle de canal et exprimer les valeurs de potentiel acquises à chacun des canaux de l'électrode à canaux multiples (110) sous la forme d'une distribution de l'impédance en deux dimensions et d'une distribution de l'impédance en trois dimensions sous un contrôle de l'ordinateur personnel (161).

10. Appareil selon l'une quelconque des revendications précédentes, dans lequel l'unité de contrôle (160) détermine une position d'un point d'acupuncture en utilisant des valeurs de potentiel numériques.

11. Méthode d'acquisition d'une impédance locale de la peau, comprenant :
(a) la disposition de deux électrodes d'une source de courant constant (130) centrées autour d'une région à mesurer sur la peau d'un patient à séparer de la région à mesurer par une distance prédéterminée et l'application d'un courant constant prédéterminé à la peau par les deux électrodes pendant une durée prédéterminée ;
(b) le positionnement d'une électrode à canaux multiples (110) parallèle à la région à mesurer et le contrôle de la pression appliquée à chacun des capteurs de mesure de l'électrode à canaux multiples (110) de manière à ce que la pression appliquée par les capteurs de mesure puisse varier ; et
(c) l'application du courant constant prédéterminé entre les deux électrodes de la source de courant constant (130) et la mesure de l'impédance de la peau dans la région à mesurer alors que le courant constant prédéterminé est appliqué.

12. Méthode selon la revendication 11, comprenant en outre :
la mesure d'une valeur de potentiel à chacun d'une pluralité de canaux compris dans une électrode à canaux multiples (110) disposée entre deux électrodes d'une source de courant constant (130) pour l'application d'un courant constant prédéterminé à la peau d'un patient par les deux électrodes ;
l'amplification et la filtration de la valeur de potentiel à chaque canal ;
la conversion de la valeur de potentiel filtrée d'un format analogique en un format numérique ; et
l'analyse de la valeur de potentiel au format numérique et l'affichage des résultats de l'analyse sous une forme d'une distribution spatiale de l'impédance en deux et trois dimensions.

13. Méthode selon la revendication 11 ou 12,
dans laquelle l'électrode à canaux multiples comprend :
une pluralité de capteurs de mesure arrangés en une forme de matrice sur une surface d'électrode ayant une aire prédéterminée.

14. Méthode selon la revendication 11, 12, ou 13, dans laquelle dans (b), la pression appliquée à chacun des capteurs de mesure est ajustée selon une courbure de la région à mesurer durant la mesure de l'impédance de la peau.

15. Méthode selon l'une quelconque des revendications 12 à 14, dans laquelle les canaux sont choisis chacun à leur tour.

16. Méthode selon l'une quelconque des revendications 11 à 15, dans laquelle le potentiel dans chaque canal est mesuré, filtré et amplifié alors que le courant constant circule.

17. Logiciel comprenant des moyens de code de logiciel adaptés pour entraîner la réalisation de toutes les étapes selon l'une quelconque des revendications 11 à 16 par l'appareil selon les revendications 1 à 10.

18. Logiciel selon la revendication 17, mis en application sur un support lisible par ordinateur.

## Patentansprüche

1. Gerät zum Messen örtlicher Hautimpedanz, umfassend:
eine Mehrkanalelektrode (110), die eine Mehrzahl von Messsensoren auf einer Elektrodenoberfläche mit einer bestimmten Flächenausdehnung aufweist;
einen Kanalselektor (120), der so angeordnet ist, dass er jeden der Kanäle in der Mehrkanalelektrode (110) in Reaktion auf ein Kanalsteuersignal auswählt;
eine Vorverarbeitungseinheit (140), die so angeordnet ist, dass sie einen an jedem der Kanäle gemessenen Potentialwert verstärkt und filtert, während der bestimmte konstante Strom durch die zu messende Region fließt;
einen Analog-Digital-Konverter (150), der so angeordnet ist, dass er den von der Vorverarbeitungseinheit (140) ausgegebenen Potentialwert in ein digitales Signal konvertiert; und **gekennzeichnet durch**:
eine konstante Stromquelle (130), die so angeordnet ist, dass sie einen bestimmten konstanten Strom in eine zu messende Region aufbringt;
eine Steuereinheit (160), die so angeordnet ist, dass sie das Kanalsteuersignal erzeugt, zum Verarbeiten des vom Analog-Digital-Konverter (150) ausgegebenen digitalen Signals und zum Steuern des gesamten Geräts; und
worin das Gerät so angeordnet ist, dass es den auf jeden der Messsensoren aufgebrachten Druck steuert, so dass der auf die Messsensoren aufgebrachte Druck verändert werden kann.

2. Gerät nach Anspruch 1, worin die Mehrzahl von Messsensoren in einer Matrixform auf der Elektrodenoberfläche angeordnet ist.

3. Gerät nach Anspruch 1 oder 2, worin die Messsensoren Stiftelektroden sind, die aus einem Metallleiter gebildet sind und eine Feder aufweisen.

4. Gerät nach einem der vorhergehenden Ansprüche, worin der Mehrkanal ferner fünfundzwanzig (25) Messsensoren umfasst, die in einer 5 x 5 Matrix angeordnet sind.

5. Gerät nach einem der vorhergehenden Ansprüche, worin die Mehrkanalelektrode (110) eine mikroelektromechanische Systemelektrode (MEMS) umfasst.

6. Gerät nach einem der vorhergehenden Ansprüche, worin die konstante Stromquelle (130) umfasst:
eine positive Elektrode (131) und eine negative Elektrode (132), so angeordnet, dass sie an einer Stelle auf der Haut zentriert um eine zu messende Region angebracht werden können, derart, dass die positive und negative Elektrode von der zu messenden Region in einem bestimmten Abstand entfernt sind, so dass der von der konstanten Stromquelle (130) ausgegebene bestimmte konstante Strom durch die positive Elektrode (131) auf die Haut aufgebracht wird, dann von der Haut durch die negative Elektrode (132) ausgegeben wird, und dann zurück in die konstante Stromquelle (130) fließt.

7. Gerät nach einem der vorhergehenden Ansprüche, worin die Vorverarbeitungseinheit (140) umfasst:
einen Differentialverstärker (141); und
einen Filter (142).

8. Gerät nach Anspruch 7, worin der Filter (142) ein Butterworth-Filter sechster Ordnung mit einer Cut-Off-Frequenz von 4 Hz oder weniger ist.

9. Gerät nach einem der vorhergehenden Ansprüche, worin die Steuereinheit (160) umfasst:
einen Personalcomputer (161) zum Steuern des Geräts; und
einen Signalprozessor (162) zum Erzeugen des Kanalsteuersignals und Ausdrücken der Potentialwerte, die an jedem der Kanäle der Mehrkanalelektrode (110) aufgenommen sind, als zweidimensionale Impedanzverteilung und dreidimensionale Impedanzverteilung unter einer Steuerung des Personalcomputers (161).

10. Gerät nach einem der vorhergehenden Ansprüche, worin die Steuereinheit (160) eine Akupunkturpunktposition unter Verwendung der digitalen Potentialwerte bestimmt.

11. Verfahren zum Erfassen einer örtlichen Hautimpedanz, umfassend:
(a) Anordnen zweier Elektroden einer konstanten Stromquelle (130) zentriert um eine zu messende Region auf der Haut eines Patienten, so dass sie von der zu messenden Region in einem bestimmten Abstand getrennt sind und Aufbringen eines bestimmten konstanten Stroms auf die Haut durch die zwei Elektroden über eine bestimmte Zeitspanne;
(b) Positionieren einer Mehrkanalelektrode (110) parallel zur zu messenden Region und Steuern des auf jeden der Messsensoren der Mehrkanalelektrode (110) aufgebrachten Drucks, so dass der von den Messsensoren ausgebrachte Druck verändert werden kann; und
(c) Aufbringen des bestimmten konstanten Stroms zwischen den zwei Elektroden der konstanten Stromquelle (130) und Messen von Hautimpedanz in der zu messenden Region, während ein bestimmter konstanter Strom aufgebracht wird.

12. Verfahren nach Anspruch 11, ferner umfassend:
Messen eines Potentialwerts an jedem einer Mehrzahl von Kanälen in einer Mehrkanalelektrode (110) angeordnet zwischen zwei Elektroden einer konstanten Stromquelle (130) zum Aufbringen eines bestimmten konstanten Stroms auf die Haut eines Patienten durch die beiden Elektroden;
Verstärken und Filtern des Potentialwerts an jedem Kanal;
Konvertieren des gefilterten Potentialwerts von einem analogen Format in ein digitales Format; und
Analysieren des Potentialwerts in digitalem Format und Anzeigen der Ergebnisse der Analyse in einer Form einer räumlichen Impedanzverteilung in zwei und drei Dimensionen.

13. Verfahren nach Anspruch 11 oder 12, worin die Mehrkanalelektrode (110) umfasst:
eine Mehrzahl vom Messsensoren, die in einer Matrixform auf einer Elektrodenfläche mit einer bestimmten Flächenausdehnung angeordnet werden.

14. Verfahren nach Anspruch 11, 12 oder 13, worin in (b) der auf jeden der Messsensoren aufgebrachte Druck in Abhängigkeit von einer Wölbung der zu messenden Region beim Messen der Hautimpedanz eingestellt wird.

15. Verfahren nach einem der Ansprüche 12 bis 14, worin die Kanäle reihum gewählt werden.

16. Verfahren nach einem der Ansprüche 11 bis 15, worin das Potential in jedem Kanal gemessen, gefiltert und verstärkt wird, während der konstante Strom fließt.

17. Computerprogramm umfassend Computerprogrammcodemittel geeignet zum Veranlassen des Geräts nach einem der Ansprüche 1-10, dass es alle Schritte nach einem der Ansprüche 11 bis 16 ausführt.

18. Computerprogramm nach Anspruch 17, ausgebildet auf einem computerlesbaren Medium.
